# EUROPÄISCHE PATENTANMELDUNG

(11) **EP 1 725 072 A2**
(43) Veröffentlichungstag der Anmeldung: **22.11.2006**
(21) Anmeldenummer: 06112566.2
(22) Anmeldetag: 12.04.2006
(51) Int. Cl.: H04R 25/00

(54) **Hörhilfevorrichtung mit zwei unterschiedlichen Ausgangswandlern und Anpassverfahren**

(30) Priorität: 15.04.2005 DE 102005017493
(71) Anmelder: Siemens Audiologische Technik GmbH, 91058 Erlangen (DE)
(72) Erfinder: Weidner, Tom, 91056 Erlangen (DE)
(74) Vertreter: Berg, Peter

(57) **Zusammenfassung**

Die Versorgung von Hörgeschädigten soll verbessert werden. Dazu ist eine Hörhilfevorrichtung vorgesehen, die über mindestens zwei Ausgangssignalwandler verfügt. So kann beispielsweise über eine gemeinsame Signalverarbeitung (SV) sowohl ein Hörer (H) als auch ein Cochlear-Implantat über eine Induktionsspule (IS) angesteuert werden. Auf diese Weise kann eine Hybriden-Versorgung an einem Ohr gewährleistet werden.

## Beschreibung

Die vorliegende Erfindung betrifft eine Hörhilfevorrichtung mit einer Signalverarbeitungseinrichtung zur Gewinnung eines Verarbeitungssignals aus einem Eingangssignal und einer Ausgangswandlereinrichtung, mit der das Verarbeitungssignal in ein Ausgangssignal wandelbar ist. Darüber hinaus betrifft die vorliegende Erfindung ein entsprechendes Verfahren zum Anpassen von Hörhilfen.

Bei zahlreichen Hörschädigungen können übliche Hörgeräte wie Hinter-dem-Ohr-Hörgeräte (HdO) oder In-dem-Ohr-Hörgeräte (I-dO) hilfreich sein. Hierzu ist jedoch Voraussetzung, dass die Cochlea (Hörschnecke) funktionsfähig ist. Ist die Cochlea jedoch ganz oder teilweise beschädigt, so dass übliche Hörgeräte keinen Hörgewinn ermöglichen, kann heute mit Hilfe eines sogenannten Cochlear-Implantats (CI), das die Funktion der Cochlea zu ersetzen versucht, der Hörnerv direkt stimuliert werden. Damit lässt sich zumindest im hochfrequenten Bereich ein Höreindruck erzielen.

Weitere Hörgerätetypen sind beispielsweise Hirnstammimplantate (ABI; Auditory Brain Stem Implants), die eingesetzt werden können, wenn der Hörnerv geschädigt ist. Darüber hinaus sind Knochenleitungshörgeräte und so genannte BAHAs (Bone Anchored Hearing Aid) zur Schallleitung über die Knochen ans Mittelohr bzw. Innenohr bekannt.

Hörschäden sind jedoch derart variantenreich, dass für einige Hörgeschädigte mit den bekannten Hörgeräten nur ein geringer Grad an Hörgewinn erzielt werden kann.

Aus der Druckschrift DE 101 14 838 A1 ist ein vollständig implantierbares Hörsystem bekannt, das eine ausgangsseitige aktorische Anordnung zum Stimulieren des Mittel- oder Innenohrs aufweist. Die aktorische Anordnung weist mindestens einen elektromechanischen Wandler zum mechanischen Stimulieren des Mittel- oder Innenohrs auf. Außerdem kann die Anordnung intracochleäre Reizelektroden zum Stimulieren des Innenohrs durch elektrische Reizung aufweisen.

Darüber hinaus ist in der Patentschrift DE 100 31 832 C1 ein Hörgerät mit einem Spracherkennungsmodul und einem Sprachsynthesemodul beschrieben. Entsprechend einer ersten Ausgestaltung wird das Ausgangssignal des Moduls einem elektroakustischen Wandler zur Schallabstrahlung in den äußeren Gehörgang zugeführt. Gemäß einer zweiten Ausgestaltung kann ein elektromechanischer Wandler vorgesehen sein, der an das Mittel-oder Innenohr angekoppelt wird.

Des Weiteren ist in der Patentschrift DE 100 18 334 C1 ein mindestens teilimplantierbares System zur Rehabilitation einer Hörstörung beschrieben. Dabei weist eine aktorische Stimulationsanordnung in Kombination einen elektromechanischen Wandler zur mechanischen Stimulation des Mittel- oder Innenohrs und ein intracochleäres, elektrisch wirkendes Reizelekrodenarray mit mindestens einer Reizelektrode zur elektrischen Stimulation des Innenohrs auf.

Die Aufgabe der vorliegenden Erfindung besteht somit darin, eine Hörhilfevorrichtung vorzuschlagen, mit der der Hörgewinn beispielsweise bei teilweise geschädigter Cochlea verbessert werden kann.

Erfindungsgemäß wird diese Aufgabe gemäß Anspruch 1 gelöst durch eine Hörhilfevorrichtung mit einer Signalverarbeitungseinrichtung zur Gewinnung eines ersten Verarbeitungssignals aus einem Eingangssignal und einer ersten Ausgangswandlereinrichtung, mit der das erste Verarbeitungssignal in ein erstes Ausgangssignal wandelbar ist, wobei mit der Signalverarbeitungseinrichtung auch ein zweites Verarbeitungssignal aus dem Eingangssignal gewinnbar ist und mindestens eine zweite Ausgangswandlereinrichtung an die Signalverarbeitungseinrichtung angeschlossen ist, mit der das zweite Verarbeitungssignal in ein zweites Ausgangssignal wandelbar ist. Das erste Ausgangssignal der erfindungsgemäßen Hörhilfevorrichtung ist ein Schallsignal und das zweite Ausgangssignal ein elektromagnetisches Signal. Damit ist es möglich, mit ein und demselben Gerät ein verstärktes Schallsignal für das übliche Luftschallhören als auch ein elektromagnetisches Steuersignal zur berührungslosen Ansteuerung eines Implantats bereitzustellen.

In vorteilhafter Weise kann durch die erfindungsgemäße Hörhilfevorrichtung eine Hybridversorgung erzielt werden, die beispielsweise die gleichzeitige Versorgung mit einem Cochlear-Implantat (oder Knochenleitungshörer) und einem nicht implantierbaren Hörgerät (Luftschall-Hörgerät oder Knochenleitungshörgerät bzw. BAHA) am selben Ohr gewährleistet. Ebenfalls möglich wäre eine kombinierte Luft- und Knochenschallversorgung. Hierzu kann die Hörhilfevorrichtung mit einem einzigen Gehäuse, einer gemeinsamen Energieversorgung sowie einer gemeinsamen Bedienung und Signalverarbeitung für die beiden Ausgangssignale ausgestattet sein. Auch die Anpasssoftware ist nun nicht mehr unabhängig, sondern integriert die verschiedenen Teile.

In der erfindungsgemäßen Hörhilfevorrichtung sind somit die Technologien von äußeren Hörgeräten und Implantaten gemeinsam genutzt, so dass ein umfassendes Hörsystem bereitgestellt werden kann, das in der Lage ist, sowohl die elektrische Stimulation als auch die "normale" Signalübertragung durch Luftschall und/oder Knochenleitung zu realisieren. Auf diese Weise kann Schwerhörigen mit sehr speziellen Hörverlusten eine verbesserte Systemlösung geboten werden.

Insbesondere kann es günstig sein, wenn mit der Signalverarbeitungseinrichtung ein Steuersignal für ein Cochlear-Implantat oder Hirnstammimplantat erzeugbar ist. Damit kann auch in Frequenzbereichen ein Höreindruck erzeugt werden, der auf dem normalen Hörweg nicht mehr erreichbar ist.

Entsprechend einer bevorzugten Ausführungsform umfasst die zweite Ausgangswandlereinrichtung eine Sendespule. Hierdurch ist beispielsweise ein Cochlear-Implantat induktiv ansteuerbar.

Entsprechend einer anderen Ausführungsvariante kann die zweite Ausgangswandlereinrichtung lösbar an der Hörervorrichtung angebracht sein. Somit ist eine Modulbauweise der Hörhilfevorrichtung realisierbar. Beispielsweise könnte so ein HdO mit einem CI-Modul oder einem BAHA-Modul je nach individuellem Bedürfnis ausgestattet werden. Auch der erste Ausgangswandler kann sich außerhalb des HdO-Gehäuses befinden, um z. B. den Luftschallwandler (Hörer) in eine Otoplastik zu integrieren.

Die Hörvorrichtung kann im Wesentlichen die äußere Form eines HdO besitzen. Somit muss ein übliches CI-Steuergerät lediglich mit einem Schallwandler und einer entsprechenden Signalverarbeitungseinheit ausgestattet werden, da diese Einheit auch die Form eines HdO besitzen kann. Die Hörhilfevorrichtung kann aber auch als Knochenleitungshörgerät oder als am Knochen verankerbares Hörgerät ausgestaltet sein. Damit lässt sich auch dieser Typ von Hörgerät beispielsweise mit einem CI kombinieren.

Die vorliegende Erfindung wird nun anhand der beigefügten Zeichnungen näher erläutert, in denen zeigen:
- FIG 1: ein erfindungsgemäßes Kombigerät entsprechend einer ersten Ausführungsform und
- FIG 2: ein erfindungsgemäßes Kombigerät entsprechend einer zweiten Ausführungsform.

Die nachfolgend näher geschilderten Ausführungsbeispiele stellen bevorzugte Ausführungsformen der vorliegenden Erfindung dar.

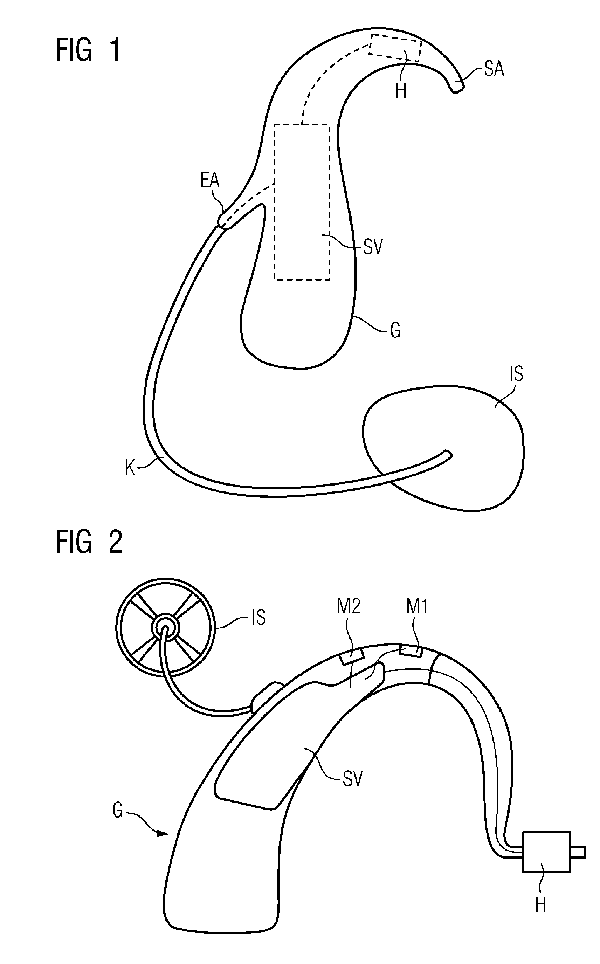

In FIG 1 ist eine erfindungsgemäße Hörhilfevorrichtung in der Form eines kombinierten HdO-CI-Hörgeräts zur Hybridenversorgung dargestellt. Es besitzt im Wesentlichen ein HdO-Gehäuse G. Dieses Gehäuse G besitzt zwei Ausgänge für zwei physikalisch verschiedene Versorgungssignale. Zum einen ist ein üblicher Schallausgang SA vorgesehen, an den ein Schallschlauch angekoppelt werden kann, um den Luftschall beispielsweise über eine Otoplastik zum Trommelfell zu leiten. Der entsprechende Schall wird von einem Hörer H generiert, der seine Signale von einer Signalverarbeitung SV erhält. Die Signalverarbeitung SV steuert jedoch nicht nur den Hörer H an, der als Schallwandler dient, sondern erzeugt auch ein elektrisches Signal, um ein Cochlear-Implantat anzusteuern. Hierzu ist an dem Gehäuse G ein zweiter Ausgang EA vorgesehen, um ein elektrisches Signal bereitzustellen. An diesen elektrischen Ausgang EA ist eine Induktionsspule IS über ein Kabel K angeschlossen. Über die Induktionsspule IS kann somit ein in der FIG nicht dargestelltes CI induktiv angesteuert werden. Dabei wird die in einen Kunststoff eingebettete Induktionsspule IS hinter dem Außenohr am Kopf des Hörgeräteträgers angebracht.

Mit dem oben dargestellten Hörgerät ist es möglich, einen Patienten mit einer Cochlea-Schädigung adäquat zu versorgen. Hierzu werden beispielsweise die niedrigen Frequenzen über das CI dem Patienten zur Verfügung gestellt, während die höheren Frequenzen, für die die Cochlea des Patienten nicht geschädigt ist, über den üblichen Schallweg mit entsprechender Verstärkung dargeboten werden können.

Bei einer alternativen Bauform kann die Induktionsspule IS auch direkt in das Gehäuse G integriert sein. Dies bedeutet, dass das Hörgerät dann annähernd die gleiche Bauform wie ein heute übliches HdO besitzt. Der Empfänger ist dann an der geeigneten Stelle zu implantieren, so dass die induktive Kopplung zum CI ausreichend hoch ist.

Die Signalverarbeitung ist vorzugsweise so ausgelegt, dass mit einer einzigen Anpasssoftware sowohl der akustische als auch der elektrische Wandler angesprochen werden kann. Damit ist zu jedem Zeitpunkt eine flexible Bereichsänderung oder Anpassung durchführbar. Insbesondere kann dadurch auf sich ändernde Hörverluste flexibler reagiert werden.

Durch die gemeinsame Signalverarbeitung für den Hörer H und den CI-Teil kann Letzterer auch von der gegenüber heute üblichen einfachen CI-Geräten weiterentwickelten Signalverarbeitung der Hörgeräte profitieren. Darüber hinaus kann auch auf eine gemeinsame Stromversorgung zurückgegriffen werden, die durch die mechanische Integration beider Geräte in ein Gehäuse erzielt wird.

Wie bereits angedeutet wurde, kann die erfindungsgemäße Hörhilfevorrichtung beliebige Arten von Ausgangswandlern besitzen. Außerdem können auch mehr als zwei, nämlich drei, vier usw., Ausgangswandler in der Hörhilfe vorgesehen sein. Damit ergeben sich spezielle Ausführungsformen wie beispielsweise ein kombiniertes Knochenleitungs-CI-Gerät, ein BAHA-CI-Gerät, ein HdO-ABI-Gerät und dergleichen.

Bei modularer Ausführungsform können die jeweiligen Hörgerätemodule beliebig aneinander gesetzt werden. Somit ist ein Wechsel oder Upgrade des einen oder anderen Teils möglich.

Die Signalverarbeitung stellt mindestens zwei verschiedene Ausgangssignale bereit. Dieses Bereitstellen kann beispielsweise zeitlich in Abhängigkeit von der jeweiligen Hörsituation oder der Frequenz des Eingangssignals abhängig gemacht werden. Beispielsweise wird das CI nur dann angesteuert, wenn die Hörsituation durch überwiegend hohe Frequenzen charakterisiert ist. Die einzelnen Komponenten des Hybrid-Hörsystems können so exklusiv oder gemeinsam angesteuert werden. Dies bedeutet, dass die Signalverarbeitung sowohl den gemeinsamen Zugriff auf beide Systeme ermöglicht als auch die flexible Bereichsaufteilung regelt.

Die Signalverarbeitungseinheit kann durch eine einzige Anpasssoftware bezüglich der Ausgangssignale der mehreren Ausgangswandler in einem einzigen Anpassvorgang angepasst werden. Dabei spielt die Anzahl der Ausgangswandler pro Gerät bei binauraler Versorgung keine Rolle.

In FIG 2 ist ein zweites Ausführungsbeispiel dargestellt. Das dort gezeigte Hörgerät verfügt grundsätzlich über die gleichen Komponenten wie das von FIG 1. Zusätzlich ist hier ein Richtmikrofon mit den beiden Mikrofonen M1 und M2 eingezeichnet. Außerdem ist zu erkennen, dass der Hörer H außerhalb des Hörgerätegehäuses G angeordnet ist.

## Patentansprüche

1. Hörhilfevorrichtung mit
- einer Signalverarbeitungseinrichtung (SV) zur Gewinnung eines ersten Verarbeitungssignals aus einem Eingangssignal und
- einer ersten Ausgangswandlereinrichtung (H), mit der das erste Verarbeitungssignal in ein erstes Ausgangssignal wandelbar ist, wobei
- mit der Signalverarbeitungseinrichtung (SV) ein zweites Verarbeitungssignal aus dem Eingangssignal gewinnbar ist und
- mindestens eine zweite Ausgangswandlereinrichtung an die Signalverarbeitungseinrichtung (SV) angeschlossen ist, mit der das zweite Verarbeitungssignal in ein zweites Ausgangssignal wandelbar ist,
**dadurch gekennzeichnet, dass**
- das erste Ausgangssignal ein Schallsignal zum Beschallen eines Trommelfells und/oder eines Knochens von außen und das zweite Ausgangssignal ein elektromagnetisches oder elektrisches Signal für ein Implantat ist.

2. Hörhilfevorrichtung nach Anspruch 1, wobei mit der Signalverarbeitungseinrichtung (SV) ein Steuersignal für ein Cochlear-Implantat oder ein Hirnstammimplantat erzeugbar ist.

3. Hörhilfevorrichtung nach einem der vorhergehenden Ansprüche, wobei die zweite Ausgangswandlereinrichtung eine Sendespule (IS) umfasst.

4. Hörhilfevorrichtung nach Anspruch 3, wobei die Sendespule (IS) in das Gehäuse der Hörhilfevorrichtung integriert ist.

5. Hörhilfevorrichtung nach einem der vorhergehenden Ansprüche, wobei die erste und/oder zweite Ausgangswandlereinrichtung lösbar an der Hörhilfevorrichtung angebracht ist.

6. Hörhilfevorrichtung nach einem der vorhergehenden Ansprüche, die im Wesentlichen die äußere Form eines Hinter-dem-Ohr-Hörgeräts besitzt.

7. Hörhilfevorrichtung nach einem der vorhergehenden Ansprüche, die als Knochenleitungshörgerät oder als am Knochen verankerbares Hörgerät ausgestaltet ist.
